Europäisches Patentamt

European Patent Office

Office européen des brevets

(19)

(11) **EP 0 993 824 A1**

(12) **DEMANDE DE BREVET EUROPEEN**

(43) Date de publication:
**19.04.2000 Bulletin 2000/16**

(51) Int Cl.⁷: **A61K 7/48**, A61K 7/02

(21) Numéro de dépôt: **99401798.6**

(22) Date de dépôt: **16.07.1999**

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **11.09.1998 FR 9811362**

(71) Demandeur: **L'OREAL**
**75008 Paris (FR)**

(72) Inventeurs:
• **Ferrari, Véronique**
  **94700 Maisons-Alfort (FR)**
• **De La Porterie, Valérie**
  **77820 Le Chatelet-en-Brie (FR)**

(74) Mandataire: **Lhoste, Catherine**
  **L'OREAL-DPI**
  **6 rue Bertrand Sincholle**
  **92585 Clichy Cédex (FR)**

(54) **Composition aqueuse comprenant un polyester sulfoné et un polyuréthane**

(57) L'invention a pour objet une composition cosmétique ou dermatologique comprenant dans une phase aqueuse:

i) un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \qquad (I)$$

dans laquelle

- A représente un groupement 1,4-phénylène, sulfo-1,3-phénylène,
- n va de 1 à 4,

- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,

la masse moléculaire en poids dudit oligomère copolyester étant inférieure à 20 000,
ii) au moins des particules de polyuréthane dispersées dans la phase aqueuse.

Cette composition est destinée au maquillage des matières kératiniques.

EP 0 993 824 A1

## EP 0 993 824 A1

**Description**

La présente invention concerne une nouvelle composition filmogène sans transfert et résistant à l'eau à application topique, comprenant un agent gélifiant hydrophile particulier de type polyester sulfoné et un polyuréthane filmogène, destinée en particulier aux domaines cosmétique et/ou dermatologique. L'invention se rapporte aussi à l'utilisation de cette composition pour le maquillage ou le traitement cosmétique des matières kératiniques d'être humain telles que la peau, les ongles, les cils, les sourcils, les cheveux, ou des muqueuses telles que les lèvres. Cette composition peut se présenter sous forme de gel, de pâte ou de produit coulé, notamment de stick.

Les produits de maquillage ou de soin de la peau ou des lèvres comme les fonds de teints ou les rouges à lèvres contiennent généralement des phases grasses telles que des cires et des huiles, des pigments et/ou des charges et, éventuellement des additifs comme des actifs cosmétiques ou dermatologiques. Elles peuvent contenir des gélifiants permettant d'obtenir la consistance souhaitée pour la composition selon son application.

Ces compositions, lorsqu'elles sont appliquées sur la peau ou les lèvres, présentent l'inconvénient de transférer, c'est-à-dire de se déposer au moins en partie, en laissant des traces, sur certains supports avec lesquels elles peuvent être mises en contact, et notamment un verre, une tasse, une cigarette, un vêtement ou la peau. Il s'ensuit une persistance médiocre du film appliqué, nécessitant de renouveler régulièrement l'application de la composition de fond de teint ou de rouge à lèvres. En outre, l'apparition de ces traces inacceptables notamment sur les cols de chemisier peut écarter certaines femmes de l'utilisation de ce type de maquillage. Il est connu de la demande EP-A-602905 d'associer des huiles volatiles à des cires pour obtenir des produits ayant des propriétés de sans-transfert améliorées. Toutefois, ces produits glissent lors de leur application sur les matières kératiniques, comme par exemple les lèvres, rendant leur application difficile à maîtriser. De plus, ces produits laissent une impression de tiraillement pendant et après leur application sur les lèvres.

Par ailleurs, les compositions après l'application sur la peau ou les lèvres peuvent s'éliminer au contact de l'eau, notamment lors de la baignade ou au contact de la pluie ou des larmes. Il est donc souhaitable de disposer de compositions présentant, en outre, de bonnes propriétés de rémanence à l'eau.

Le but de la présente invention est de proposer une nouvelle composition filmogène aqueuse ne présentant pas les inconvénients évoqués ci-dessus et présentant notamment des propriétés de sans transfert total, de rémanence à l'eau, d'absence de tiraillement pendant et après l'application sur les matières kératiniques, et d'application facile.

Les inventeurs ont découvert qu'une telle composition pouvait être obtenue en utilisant un agent gélifiant hydrophile particulier de type polyester sulfoné et un polyuréthane filmogène en dispersion aqueuse. On obtient alors une composition laissant après l'application un film présentant une bonne tenue, ne transférant pas du tout, et résistant à l'eau. Le film est notamment brillant, non collant et confortable (non tiraillement) pendant et après l'application de la composition sur les matières kératiniques. Le film produit également une sensation de fraîcheur et est facile à démaquiller avec les démaquillants habituellement utilisés. De plus, la composition est moins glissante que les produits sans transfert contenant des huiles volatiles, facilitant ainsi son application sur les matières kératiniques.

La présente invention a donc pour objet une nouvelle composition filmogène à application topique comprenant dans une phase aqueuse un agent gélifiant hydrophile de type polyester sulfoné, caractérisée par le fait que l'agent gélifiant hydrophile est un oligomère copolyester téréphtalique hydrosoluble ou hydrodispersible et que la composition comprend également des particules de polyuréthane dispersées dans la phase aqueuse.

Les agents gélifiants particuliers utilisés selon l'invention sont des oligomères copolyesters téréphtaliques hydrosolubles ou hydrodispersables comprenant essentiellement des motifs répétitifs dicarboxylates de formule (I):

$$[-CO-A-CO-O-(CH_2-CH_2O)_n-] \qquad (I)$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,

la masse moléculaire en poids desdits oligomères copolyesters étant inférieure à 20 000, de préférence inférieure à 15 000.

De manière préférentielle, au moins 40 % en mole, et plus préférentiellement entre 40 et 90 % en mole des motifs de formule (I) sont des motifs de formule (I) pour lesquels A représente un groupement 1,4-phénylène et n est égal à 1. De préférence, au moins 10 % en mole, plus préférentiellement entre 10 % et 25 % en mole des motifs de formule (I) sont des motifs de formule (I) pour lesquels A représente un groupement sulfo-1,3-phénylène.

Les extrémités des chaînes desdits oligomères copolyesters peuvent être semblables ou différentes et représentées essentiellement par les groupements de formule (I'):

$$-CO-A-CO-O-(CH_2-CH_2-O)_n-H \qquad (I')$$

dans laquelle A et n sont définis ci-dessus.

Lesdits oligomères peuvent également présenter en extrêmités de chaîne, et en quantité mineure, des groupements de formules

$$-A-CO-OH$$

$$-A-CO-OR$$

formules dans lesquelles A est défini ci-dessus et R représente un groupement alkyle en $C_1$-$C_4$.

Lorsque A représente un groupement sulfo-1,3-phénylène, il s'agit plus particulièrement d'un sulfonate de métaux alcalins, notamment de sodium ou de potassium, ou d'un sulfonate d'ammonium ou de mono-, di-, tri- ou tétra-alkylammonium inférieur. Par alkylammonium inférieur, on entend de préférence un ammonium dont le ou les radicaux alkyles sont des alkyles inférieurs, de préférence en $C_1$-$C_6$. De manière préférentielle, il s'agit d'un sulfonate de sodium.

L'oligomère copolyester peut comprendre éventuellement jusqu'à 20 % en mole, de préférence de 0,5 à 5 % en mole de motifs de formule (I) pour lesquels A représente un groupement 1,3-phénylène.

Selon un mode de réalisation préférentiel de l'invention, l'oligomère copolyester ci-dessus a une masse moléculaire en poids allant de 5 000 et 14 000, et plus préférentiellement de 8 000 à 10 000. Les masses moléculaires en poids sont mesurées par chromatographie par perméation de gel dans le diméthylacétamide contenant $10^{-2}$ N de LiBr, à 100°C. Les résultats sont exprimés en équivalents polystyrène.

Lesdits oligomères copolyesters peuvent être obtenus par les procédés usuels de préparation des polyesters par voie fondue, voie solvant ou voie interfaciale, procédés faisant intervenir des réactions

- . d'estérification de diacides et de diols et polycondensation
- . de transestérification de diesters et de diols et polycondensation
- . d'autocondensation d'hydroxyacides
- . de Schotten-Baumann par mise en oeuvre de diols et de chlorures d'acide et polycondensation
- . de polymérisation de lactones

en contrôlant la teneur minimum en motifs de formule (I) pour lesquels A représente un groupement 1,4-phénylène et n est égal à 1, semblables par les rapports stoechiométriques initiaux des différents monomères et par la maîtrise des réactions secondaires.

Un mode de préparation particulièrement intéressant est celui par transestérification / polycondensation et / ou d'estérification / polycondensation par voie fondue à l'aide d'un catalyseur de transestérification et/ou estérification.

La maîtrise de la structure est obtenue par contrôle de la teneur minimum en motifs de formule (I) pour lesquels A représente un groupement 1,4-phénylène et n est égal à 1, semblables par les rapports stoechiométriques initiaux des différents monomères diacides et/ou diesters et diol et par mise en oeuvre d'un agent limiteur d'éthérification, agent limiteur qui peut être un composé basique tel que les amines aliphatiques ou aromatiques ou un hydroxyde ou acétate de métaux alcalins ou alcalino-terreux.

Le contrôle de la masse moléculaire est obtenu d'une manière connue de l'homme du métier, par compromis adéquat entre la pression, la température et le temps.

Les oligomères copolyesters téréphtaliques utilisés selon l'invention peuvent être préparés par estérification et/ou transestérification / polycondensation d'une composition monomère à base:

- d'acide, anhydride ou diester téréphtalique (Tp)
- d'acide, anhydride ou diester sulfoisophtalique (SIp)
- éventuellement d'acide, anhydride ou diester isophtalique (Ip)

- et d'éthylène glycol (EG)

selon des quantités relatives correspondant à

* un rapport molaire (Slp) / [(Tp)+(Slp)+(lp)] d'au moins 7/100, de préférence d'au moins 10/100, tout particulièrement de 10/100 à 25/100
* un rapport molaire (lp) / [(Tp)+(Slp)+(lp)] de 20/100 au plus, de préférence de 5/100 au plus
* un rapport molaire (EG) / [(Tp)+(Slp)+(lp)] de 2/1 à 3/1.

en présence d'un catalyseur d'estérification et/ou transestérification et d'un limiteur de d'éthérification.

Le monomère téréphtalique (Tp) est de préférence mis en oeuvre sous la forme de diester inférieur (diester de dialkyle en $C_1$-$C_4$), de diméthyle de préférence.

Le monomère sulfoisophtalique (Slp) est de préférence mis en oeuvre sous la forme d'un sulfonate de métal alcalin (sodium notamment) de diester inférieur (d'alkyle en $C_1$-$C_4$), de méthyle de préférence. On peut citer tout particulièrement le sodio-oxysulfonyl-5 isophtalate de diméthyle.

Le monomère isophtalique (lp) éventuel est de préférence mis en oeuvre sous la forme d'acide isophtalique.

Lorsque tous les monomères "diacides" sont mis en oeuvre sous la forme d'un diesters, l'opération de transestérification (interéchange) entre ces monomères "diacides" et l'éthylène glycol est effectuée à une température supérieure ou égale à 130°C, de préférence de l'ordre 140 à 220°C et tout particulièrement de l'ordre de 180 à 220°C ; à cette température le méthanol (cas préférentiel des diesters méthyliques) formé est éliminé du milieu réactionnel de préférence par distillation. Cette opération d'interéchange est réalisée en présence d'un catalyseur de transestérification métallique et d'un limiteur d'éthérification. Ledit catalyseur est de préférence un carboxylate métallique, tel que l'acétate de manganèse, l'acétate de zinc, l'acétate de cobalt ou l'acétate de calcium, ou d'un titanate organique ou minéral, tel que le titanate de butyle, le titanate de nitrilo-2,2',2''-triéthyle (ou aminotriéthanolate de titane jouant en outre le rôle de limiteur d'éthérification) ou le titanate de calcium. Les catalyseurs préférés sont les titanates organiques ; ils sont mis en oeuvre en quantités de l'ordre d'au moins 0,001% en poids exprimé en titane, de préférence de l'ordre de 0,002% à 0,02% en poids de titane par rapport au poids de réactifs présents.

L'agent limiteur d'éthérification peut être un composé basique tel que les amines aliphatiques ou aromatiques (triéthanolamine, carbonate de guanidine, diméthylaniline, naphtylamine ...) ou un hydroxyde ou acétate de métaux alcalins ou alcalino-terreux (acétate de sodium, potassium, benzoate de sodium ...). Il est mis en oeuvre généralement en quantité de l'ordre de 0,001% à 0,05% par rapport au poids de réactifs présents.

La durée de l'opération d'interéchange est de 1 à 4 heures ; elle est généralement de l'ordre de 2 à 3 heures.

Lorsque plus de 90% de la quantité théorique de méthanol a été distillée, le polyol excédentaire est éliminé en portant la température du milieu réactionnel à 230°C.

L'opération de polycondensation est de préférence réalisée à une température de l'ordre de 230 à 280°C, de préférence de l'ordre de 240 à 260°C, dans un autre réacteur préalablement porté à cette température et progressivement mis sous vide jusqu'à une pression qui peut aller jusqu'à 10 Pa ; une réduction de pression jusqu'à 10 millibar environ dure de l'ordre de 40 minutes.

L'opération de polycondensation se déroule avec élimination de molécules de polyol, cette opération est stoppée lorsque le couple moteur de l'arbre d'agitation indique une valeur équivalente à environ 0,5 à 5 mètres.newton pour une température de 250°C de la masse réactionnelle et une vitesse d'agitation de 80 tours / minute d'un mobile en forme d'ancre dans un réacteur de 7,5 litres. Le vide est ensuite cassé à l'azote, et le polymère est coulé dans une lingotière ; après refroidissement, le polymère est broyé.

Lorsque l'un des monomères "diacides" est présent sous forme diacide ou anhydride et le ou les autres sous forme de diester(s), lesdits oligomères copolyesters sont obtenus en réalisant d'abord une opération de transestérification des monomères diesters avec de l'éthylène glycol dans les conditions ci-dessus décrites, suivie d'une opération d'estérification dans le milieu du monomère diacide ou anhydride avec de l'éthylène glycol, puis polycondensation dans les conditions décrites ci-dessus, la quantité totale d'éthylène glycol étant répartie entre les deux opérations (transestérification et estérification).

Si nécessaire, l'opération d'estérification est réalisée par ajout dans le milieu réactionnel résultant de l'opération de transestérification, du monomère sous forme diacide ou anhydride et d'éthylène glycol préalablement mis en suspension, à une température correspondant à celle de la fin de la température d'interéchange ; la période d'introduction est de l'ordre de 1 heure.

Cette opération d'estérification est réalisée à une température de l'ordre de 230 à 280°C, de préférence de l'ordre de 250 à 260°C, en présence d'un catalyseur du même type que celui de transestérification et d'un agent limiteur d'éthérification.

L'opération est réalisée en présence des mêmes types de catalyseur et de limiteur d'éthérification que ceux mis en oeuvre lors de l'opération de transestérification, et ce dans les mêmes proportions.

La réaction s'effectue avec élimination d'eau qui est soutirée du réacteur en même temps que le polyol en excès.

Ce type de procédé de préparation est notamment décrit dans la demande de brevet WO 95/32997.

L'oligomère copolyester téréphtalique peut être présent dans la composition selon l'invention en une teneur allant de 0,5 % à 40 % en poids, par rapport au poids total de la composition. Selon la forme recherchée, gélifiée, pâteuse ou solide, on emploiera des quantités différentes de gélifiant particulier. Pour une composition solide, on emploiera de préférence de 10 à 40 % en poids de gélifiant particulier, plus préférentiellement de 15 à 30 % en poids. Pour une composition gélifiée ou pâteuse, on emploiera de préférence de 0,5 à 10 % en poids de gélifiant particulier, plus préférentiellement de 2 à 5 % en poids.

Le polyuréthane en dispersion aqueuse présent dans la composition selon l'invention peut être un polyuréthane anionique. Ce caractère anionique est notamment dû à la présence de groupements à fonction acide carboxylique ou sulfonique dans le polymère.

Selon l'invention, on peut utiliser une ou plusieurs dispersions aqueuses d'un ou plusieurs polyuréthanes.

Le polyuréthane peut être choisi parmi les polyester-polyuréthanes et les polyéther-polyuréthanes, et de préférence parmi les polyester-polyuréthanes anioniques.

La taille des particules de polyuréthane peut aller de 2 à 300 nm, et de préférence de 2 à 150 nm.

Avantageusement, le polyuréthane en dispersion aqueuse peut être être choisi parmi ceux dont la dureté d'un film obtenu après séchage, durant 24 heures à 30 °C et à 50 % d'humidité relative, d'une couche de 300 µm d'épaisseur (avant séchage) d'une dispersion aqueuse à 28 % de matière sèche desdites particules de polyuréthane va de 10 à 180 secondes, et mieux de 40 à 150 secondes. La dureté du film de polymère est mesurée selon la norme ASTM D-43-66, ou la norme NF-T 30-016 (octobre 1981), à l'aide d'un pendule de Persoz.

On a constaté que les polyuréthanes en dispersion aqueuse dont le film de polymère présente une dureté allant de 40 à 150 secondes, comme défini précédemment, conviennent particulièrement bien lorsque le composition se présente sous la forme solide, notamment lorsque la teneur en oligomère copolyester téréphtalique va de 10 à 40 % en poids, par rapport au poids total de la composition. En effet, ces dispersions aqueuses de polyuréthane permettent d'obtenir une composition sous forme solide, par exemple en stick ou en coupelle, sans diminution de la dureté du stick ou de la coupelle par rapport à une composition solide analogue ne contenant que l'oligomère copolyester téréphtalique. La composition solide selon l'invention permet également, après son application, la formation d'un film sur les matières kératiniques ayant de bonnes propriétés de sans-transfert et de résistance à l'eau.

Comme polyuréthane utilisable selon l'invention, on peut notamment citer les polyester-polyuréthanes vendus sous les dénominations "AVALURE UR-405®", "AVALURE UR-410®", "AVALURE UR-425®", "SANCURE 2060®" par la société GOODRICH et les polyéther-polyuréthanes vendus sous les dénominations "SANCURE 878®" par la société GOODRICH, "NEOREZ R 970®" par la société ICI.

Selon l'invention, le polyuréthane peut être présent dans la composition en une quantité en matières sèches allant de 1 % à 40 % en poids par rapport au poids total de la composition, et de préférence de 5 % à 20 % en poids.

Avantageusement, l'oligomère copolyester téréphtalique et le polyuréthane peuvent être présents dans la composition selon l'invention dans un rapport pondéral oligomère/polyuréthane allant de 0,5 à 20, et de préférence de 1 à 5.

Par ailleurs, la composition selon l'invention peut contenir des adjuvants couramment utilisés dans les compositions cosmétiques, notamment topiques. On peut citer à titre d'exemple d'adjuvants les colorants, les pigments, les nacres, les agents anti-UV, les conservateurs, les agents épaississants, les agents plastifiants, les tensioactifs, les cires, les huiles, les parfums, les agents modificateurs de pH, les agents hydratants. Bien entendu, l'homme du métier veillera à choisir ce ou ces éventuels adjuvants, et/ou leur quantité, de telle manière que les propriétés avantageuses de la composition selon l'invention ne soient pas, ou substantiellement pas, altérées par l'adjonction envisagée.

La composition selon l'invention peut être utilisée pour le maquillage, le soin ou le traitement cosmétique non thérapeutique des matières kératiniques et/ou des muqueuses. La composition de maquillage peut être un produit de maquillage des lèvres, notamment sous forme de pâte ou de stick, un fond de teint, un fard à paupières ou à joue, un mascara pour les cils ou les cheveux, un eye-liner, un anti-cernes, ou bien encore une composition de maquillage du corps. La composition de traitement ou de soin cosmétique peut être une composition pour le soin du visage, du cou, des mains, du corps, des ongles ou des lèvres, une composition déodorante ou encore de protection solaire.

L'invention se rapporte également à un procédé non thérapeutique de traitement ou de maquillage des matières kératiniques et/ou des muqueuses consistant à appliquer sur les matières kératiniques et/ou des muqueuses une composition telle que décrite précédemment.

L'invention a également pour objet l'utilisation d'un oligomère copolyester téréphtalique et d'un polyuréthane en dispersion aqueuse tels que définis précédemment, dans une composition cosmétique ou dermatologique pour diminuer, voire supprimer, le transfert du film de cette composition déposé sur les matières kératiniques et/ou les muqueuses et/ou pour supprimer le dépôt de traces sur un autre support autre que lesdites matières kératiniques et/ou lesdites muqueuses.

L'invention a aussi pour objet l'utilisation d'un oligomère copolyester téréphtalique et d'un polyuréthane en dispersion aqueuse tels que définis précédemment dans une composition cosmétique ou dermatologique pour l'obtention d'un film de cette composition résistant à l'eau et/ou frais et/ou confortable.

L'invention a encore pour objet l'utilisation d'une composition telle que définie précédemment pour l'obtention d'un film de cette composition résistant à l'eau et/ou ayant des propriétés de non transfert et/ou frais et/ou confortable.

Un autre objet de l'invention est l'utilisation d'un polyuréthane en dispersion aqueuse tel que défini précédemment dans une composition comprenant dans une phase aqueuse un oligomère copolyester téréphtalique tel que défini précédemment pour obtenir un produit solide stable résistant à l'eau.

On va maintenant donner des exemples illustrant la présente invention sans toutefois la limiter.

**Exemple 1      Préparation d'un oligomère copolyester téréphtalique**

Dans un réacteur en acier inoxydable de 7,5 litres, muni d'un agitateur à ancre tournant à 80 tr/mn relié à un couplemètre KYOWA, d'une double enveloppe pour la circulation d'un liquide caloporteur et d'une colonne à distiller régulée par une électrovanne on introduit:

- 11,47 moles de téréphtalate de diméthyle
- 2,53 moles de diméthylisophtalate-5 sulfonate de sodium
- 39,16 moles d'éthylène glycol
- 54 ppm en poids de titane, sous forme d'aminotriéthanolate de titane comme catalyseur et agent limiteur d'éthérification.

Le mélange est préchauffé à 180°C. Il est ensuite porté jusqu'à la température de 220°C en environ 130 minutes, pour distiller plus de 90% de la quantité théorique de méthanol.

Le mélange réactionnel est ensuite amené à 230°C en 30 minutes. Lorsque la masse réactionnelle a atteint cette température, on introduit en 60 minutes, toujours à 230°C, une suspension dont la composition est la suivante :

- 0,5 mole d'acide isophtalique
- 2,36 moles d'acide téréphtalique
- 8 moles d'éthylène glycol

La masse réactionnelle est alors amenée à la température de 250°C en 60 minutes.

Pendant la période d'introduction du mélange et pendant la période de chauffage jusqu'à 250°C, on distille un mélange d'eau et d'éthylène glycol sans rétrogradation.

Le mélange réactionnel est ensuite transféré dans un autoclave préchauffé à 250°C puis mis sous pression réduite de 100 millibar en 22 minutes. Après 2 minutes dans ces conditions de température et de pression, la masse réactionnelle est coulée et refroidie.

Le copolyester obtenu présente les caractéristiques structurales décrites au tableau 1 ci-après, dans lequel:

\* "% molaire des motifs diacides" correspond à la teneur, en %, de chaque diacide ou diester mise en oeuvre par rapport à l'ensemble des diacides ou diesters mis en oeuvre.

"Tp" signifie : motif téréphtalique
"Ip" signifie : motif isophtalique
"SIp" signifie : motif sulfoisophtalique
    Les caractéristiques de la partie "glycol" des copolyesters sont obtenues par méthanolyse des produits à 190°C pendant 16 heures suivies d'une analyse par la technique de chromatographie en phase vapeur et dosage par étalonnage interne.

- "% molaire des motifs diols" correspond à la teneur, en %, des motifs oxyéthylène "G", des motifs di(oxyéthylène) "2G", des motifs tri(oxyéthylène) "3G" et des motifs tetra(oxyéthylène) "4G", par rapport à l'ensemble des motifs diols.

\* "%GT/$\Sigma$ motifs" correspond au % molaire des motifs de formule (I)

$$[-CO-A-CO-O-(CH_2-CH_2-O)_n-] \tag{I}$$

où A est 1,4 phénylène et n=1

par rapport à l'ensemble des motifs de formule (I)

où A est 1,4 phénylène, sulfo 1,3 phénylène et éventuellement 1,3 phénylène et n va de 1 à 4

"%GT/Σ motifs" se calcule par la formule suivante :

$$\text{\%GT/}\Sigma \text{ motifs} = (\text{\% molaire de motifs Tp}) \times (\text{\% molaire de motifs G}) / 100$$

\* La masse molaire des polyesters (Mw) est déterminée par chromatographie par permation de gel (GPC) dans le DMAc/LiBr à 100%, les résultats sont données en équivalents polystyrène.

| % molaire des motifs diacides | |
|---|---|
| Tp | 82 |
| Ip | 3 |
| Slp | 15 |
| %GT/Σ motifs | 46,5 |
| % molaire des motifs diols | |
| G | 56,8 |
| 2G | 30,7 |
| 3G | 10 |
| 4G | 2,5 |
| Mw | 8 000 |

**Exemple 2 :**

On a préparé un rouge à lèvres en forme de stick ayant la composition suivante :

- oligomère copolyester téréphtalique de l'exemple 1    20 %
- dispersion aqueuse de polyester-polyuréthane à 35 % de matières sèches (Avalure UR-405 de GOODRICH)    10 % MA
- pigments    5 %
- propylène glycol    2,5 %
- eau    qsp    100 %

On obtient un rouge à lèvres permettant l'obtention d'un film "sans transfert" total et qui résiste parfaitement bien à l'eau.

**Exemple 3:**

On a préparé un rouge à lèvres en forme de stick ayant la composition suivante :

- oligomère copolyester téréphtalique de l'exemple 1    20 %
- dispersion aqueuse de polyester-polyuréthane à 49 % de matières sèches (Avalure UR-425 de GOODRICH)    -    10 % MA
- pigments    5 %
- propylène glycol    2,5 %
- eau    qsp    100 %

On obtient un rouge à lèvres permettant l'obtention d'un film "sans transfert" total et qui résiste parfaitement bien à l'eau.

**Exemple 4 :**

On a préparé un eye-liner en forme de gel ayant la composition suivante :

- oligomère copolyester téréphtalique de l'exemple 1     10 %
- dispersion aqueuse de polyester-polyuréthane à 49 % de matières sèches (Avalure UR-425 de GOODRICH)
     8 % MA
- pigments noirs     5 %
- propylène glycol     4 %
- eau     qsp     100 %

On obtient un eye-liner permettant l'obtention d'un maquillage "sans transfert" et qui résiste parfaitement bien à l'eau.

### Exemple 5 :

On a préparé un produit de maquillage du corps ayant la composition suivante :

- oligomère copolyester téréphtalique de l'exemple 1     5 %
- dispersion aqueuse de polyester-polyuréthane à 49 % de matières sèches (Avalure UR-405 de GOODRICH)
     5 % MA
- pigments     5 %
- propylène glycol     2,5 %
- eau     qsp     100 %

## Revendications

1. Composition filmogène à application topique comprenant dans une phase aqueuse un agent gélifiant hydrophile de type polyester sulfoné, caractérisée en ce que :

   i) le gélifiant hydrophile de type polyester sulfoné est un oligomère copolyester téréphtalique hydrosoluble ou hydrodispersable comprenant essentiellement des motifs répétitifs dicarboxylates de formule (I):

   $$[-CO-A-CO-O-(CH_2-CH_2O)_n-] \tag{I}$$

   dans laquelle

   - A représente un groupement 1,4-phénylène, sulfo-1,3-phénylène et éventuellement 1,3-phénylène,
   - n va de 1 à 4,
   - au moins 35 % en mole desdits motifs de formule (I) étant des motifs pour lesquels A représente un groupement 1,4-phénylène et n est égal à 1,
   - au moins 7 % en mole desdits motifs de formule (I) étant des motifs pour lesquels A représente un groupement sulfo-1,3-phénylène,
   - et éventuellement jusqu'à 20 % en mole, de préférence jusqu'à 0,5 % à 5 % en mole de motifs de formule (I) pour lesquels A représente un groupement 1,3-phénylène, et

   la masse moléculaire en poids dudit oligomère copolyester étant inférieure à 20 000,
   ii) et que la composition comprend également au moins des particules de polyuréthane dispersées dans la phase aqueuse.

2. Composition selon la revendication 1, caractérisée par le fait que l'oligomère copolyester a une masse moléculaire en poids inférieure à 15 000.

3. Composition selon la revendication 1 ou 2, caractérisée par le fait que l'oligomère copolyester téréphtalique a une masse moléculaire en poids allant de 5 000 à 14 000, et mieux de 8 000 à 10 000.

4. Composition selon l'une quelconque des revendications précédentes, caractérisée par le fait que l'oligomère copolyester téréphtalique comprend au moins 40 % en mole desdits motifs de formule (I) pour lesquels A représente un groupement 1,4-phénylène et n est égal à 1.

**5.** Composition selon l'une quelconque des revendications précédentes, caractérisée par le fait que l'oligomère copolyester téréphtalique comprend de 40 à 90 % en mole desdits motifs de formule (I) pour lesquels A représente un groupement 1,4-phénylène et n est égal à 1.

**6.** Composition selon l'une quelconque des revendications précédentes, caractérisée par le fait que l'oligomère copolyester téréphtalique comprend au moins 10 % en mole des motifs de formule (I) pour lesquels A représente un groupement sulfo-1,3-phénylène.

**7.** Composition selon l'une quelconque des revendications précédentes, caractérisée par le fait que l'oligomère copolyester téréphtalique comprend de 10 % et 25 % en mole desdits motifs de formule (I) pour lesquels A représente un groupement sulfo-1,3-phénylène.

**8.** Composition selon l'une quelconque des revendications précédentes, caractérisée par le fait que l'oligomère copolyester téréphtalique comprend jusqu'à 20 % en mole, et de préférence de 0,5 % à 5 % en mole, de motifs de formule (I) pour lesquels A représente un groupement 1,3-phénylène.

**9.** Composition selon l'une quelconque des revendications précédentes, caractérisée par le fait que l'oligomère copolyester téréphtalique est présent en une teneur allant de 0,5 % à 40 % en poids, par rapport au poids total de la composition.

**10.** Composition selon l'une quelconque des revendications précédentes sous forme solide, caractérisée par le fait que l'oligomère copolyester téréphtalique est présent en une teneur allant de 10 % à 40 % en poids, par rapport au poids total de la composition, et mieux de 15 % à 30 % en poids.

**11.** Composition selon l'une quelconque des revendications 1 à 9 sous forme de gel, caractérisée par le fait que l'oligomère copolyester téréphtalique est présent en une teneur allant de 0,5 % à 10 % en poids, par rapport au poids total de la composition, et mieux de 2 % à 5 % en poids.

**12.** Composition selon l'une quelconque des revendications précédentes, caractérisée par le fait que le polyuréthane est un polyuréthane anionique.

**13.** Composition selon l'une quelconque des revendications précédentes, caractérisée par le fait que le polyuréthane est choisi parmi les polyester-polyuréthanes et les polyéther-polyuréthanes.

**14.** Composition selon l'une quelconque des revendications précédentes, caractérisée par le fait que le polyuréthane est un polyester-polyuréthane anionique.

**15.** Composition selon l'une quelconque des revendications précédentes, caractérisée par le fait que les particules de polyuréthane sont telles que la dureté d'un film obtenu après séchage, durant 24 heures à 30 °C et à 50 % d'humidité relative, d'une couche de 300 μm d'épaisseur d'une dispersion aqueuse à 28 % de matière sèche desdites particules va de 10 à 180 secondes, et mieux de 40 à 150 secondes.

**16.** Composition selon l'une quelconque des revendications précédentes, caractérisée par le fait que les particules de polyuréthane ont une taille allant de 2 à 300 nm.

**17.** Composition selon l'une quelconque des revendications précédentes, caractérisée par le fait que le polyuréthane est présent en une quantité en matières sèches allant de 3 % à 50 % en poids par rapport au poids total de la composition.

**18.** Composition selon l'une quelconque des revendications précédentes, caractérisée par le fait que l'oligomère copolyester téréphtalique et le polyuréthane sont présents selon un rapport pondéral oligomère/polyuréthane allant de 0,5 à 20, et mieux de 1 à 5.

**19.** Composition selon l'une quelconque des revendications précédentes, caractérisée par le fait que la composition est une composition cosmétique ou dermatologique.

**20.** Composition cosmétique selon l'une quelconque des revendications précédentes, caractérisée par le fait qu'elle comprend au moins un adjuvant choisi dans le groupe formé par les colorants, les pigments, les nacres, les agents anti-UV, les conservateurs, les agents épaississants, les agents plastifiants, les tensioactifs, les cires, les huiles, les parfums, les agents modificateurs de pH, les agents hydratants.

21. Composition selon l'une quelconque des revendications précédentes, caractérisée par le fait qu'elle se présente sous forme de composition de maquillage ou de traitement cosmétique des matières kératiniques et/ou muqueuses.

22. Composition selon l'une quelconque des revendications précédentes, caractérisée par le fait que la composition se présente sous la forme d'un produit de maquillage des lèvres, d'un fond de teint, d'un fard à paupières ou à joue, d'un mascara, d'un eye-liner, d'un anti-cernes, d'une composition de maquillage du corps, d'une composition pour le soin du visage, du cou, des mains, du corps, des ongles ou des lèvres, d'une composition déodorante, d'une composition de protection solaire.

23. Procédé non thérapeutique de maquillage ou de traitement des matières kératiniques et/ou des muqueuses, caractérisé par le fait que l'on applique sur les matières kératiniques et/ou des muqueuses une composition selon l'une des revendications 1 à 22.

24. Utilisation d'un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
et d'un polyuréthane en dispersion aqueuse, dans une composition cosmétique ou dermatologique pour diminuer, voire supprimer, le transfert du film de cette composition déposé sur les matières kératiniques et/ou les muqueuses et/ou pour supprimer le dépôt de traces sur un autre support autre que lesdites matières kératiniques et/ou lesdites muqueuses.

25. Utilisation d'un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \tag{I}$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
et d'un polyuréthane en dispersion aqueuse dans une composition cosmétique ou dermatologique pour l'obtention d'un film de cette composition résistant à l'eau et/ou frais et/ou confortable.

**26.** Utilisation d'une composition selon l'une quelconque des revendications 1 à 22 pour l'obtention d'un film de cette composition résistant à l'eau et/ou ayant des propriétés de non transfert et/ou frais et/ou confortable.

**27.** Utilisation d'un polyuréthane en dispersion aqueuse dans une composition comprenant dans une phase aqueuse un oligomère copolyester téréphtalique hydrosoluble ou hydrodispersable comprenant essentiellement des motifs répétitifs dicarboxylates de formule (I):

$$[-CO-A-CO-O-(CH_2-CH_2O)_n-] \qquad\qquad (I)$$

dans laquelle

- A représente un groupement 1,4-phénylène, sulfo-1,3-phénylène et éventuellement 1,3-phénylène,
- n va de 1 à 4,
- au moins 35 % en mole desdits motifs de formule (I) étant des motifs de formule (I) pour lesquels A représente un groupement 1,4-phénylène et n est égal à 1,
- au moins 7 % en mole desdits motifs de formule (I) étant des motifs de formule (I) pour lesquels A représente un groupement sulfo-1,3-phénylène,
- et éventuellement jusqu'à 20 % en mole, de préférence jusqu'à 0,5 % à 5 % en mole de motifs de formule (I) pour lesquels A représente un groupement 1,3-phénylène, et

la masse moléculaire en poids dudit oligomère copolyester étant inférieure à 20 000,
pour obtenir un produit solide stable résistant à l'eau.

**Office européen des brevets** **RAPPORT DE RECHERCHE EUROPEENNE**

Numéro de la demande

EP 99 40 1798

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | GB 2 238 242 A (MAYBE HOLDING) 29 mai 1991 (1991-05-29)<br><br>* revendications 1,3,9 *<br>* page 5, ligne 23-26 *<br>* page 6, ligne 8 - page 7, ligne 33 *<br>* page 9, ligne 1-30 *<br>* page 10, ligne 31,32 *<br>----- | 1,2,7,9, 11,19, 20,22-27 | A61K7/48 A61K7/02 |

DOMAINES TECHNIQUES RECHERCHES (Int.Cl.7)

A61K

Le présent rapport a été établi pour toutes les revendications

| Lieu de la recherche | Date d'achèvement de la recherche | Examinateur |
|---|---|---|
| LA HAYE | 1 décembre 1999 | Peeters, J |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 99 40 1798

La présente annexe indique les membres de la famille de brevets relatifs aux documents brevets cités dans le rapport de recherche européenne visé ci−dessus.
Lesdits members sont contenus au fichier informatique de l'Officeeuropéen des brevets à la date du
Les renseignements fournis sont donnés à titre indicatif et n'engagent pas la responsabilité de l'Office européen des brevets.

01−12−1999

| Document brevet cité au rapport de recherche | Date de publication | Membre(s) de la famille de brevet(s) | Date de publication |
|---|---|---|---|
| GB 2238242 A | 29−05−1991 | US 5053221 A | 01−10−1991 |
| | | CA 2030289 A | 21−05−1991 |
| | | JP 2814018 B | 22−10−1998 |
| | | JP 3209308 A | 12−09−1991 |
| | | MX 172080 B | 01−12−1993 |

EPO FORM P0460

Pour tout renseignement concernant cette annexe : voir Journal Officiel de l'Office européen des brevets, No.12/82